# EUROPEAN PATENT APPLICATION

(11) **EP 2 298 327 A1**
(43) Date of publication of application: **23.03.2011**
(21) Application number: 08874479.2
(22) Date of filing: 26.12.2008
(51) Int. Cl.: A61K 33/24, A61K 9/10, A61K 39/395, A61K 41/00, A61K 47/10, A61K 47/12, A61K 47/18, A61K 47/32, A61K 47/34, A61K 47/48, A61P 35/00

(54) **ANTI-TUMOR AGENT**

(30) Priority: 29.05.2008 JP 2008140585; 07.08.2008 JP 2008204114
(71) Applicant: Toto Ltd., Fukuoka 802-8601 (JP)
(72) Inventor: KANEHIRA, Koki, Kitakyushu-shi Fukuoka 802-8601 (JP); SONEZAKI, Shuji, Kitakyushu-shi Fukuoka 802-8601 (JP); OGAMI, Yumi, Kitakyushu-shi Fukuoka 802-8601 (JP); NAKAMURA, Tomomi, Kitakyushu-shi Fukuoka 802-8601 (JP); BANZAI, Toshiaki, Kitakyushu-shi Fukuoka 802-8601 (JP)
(74) Representative: ABG Patentes, S.L.
(86) International application number: PCT/JP2008/004011
(87) International publication number: WO 2009/144775

(57) **Abstract**

Titanium oxide-antibody conjugated particles are disclosed, which are provided with selective binding ability without loss of dispersibility and catalytic activity by modifying titanium oxide conjugated particles, dispersed in a water-based solvent by a water-soluble polymer, with an antibody via a linker molecule bound without changing the nature of the water-soluble polymer. The present invention is an antitumor agent, comprising titanium oxide-antibody conjugated particles, wherein a linker molecule is bound to the titanium oxide surface of the titanium oxide conjugated particles, dispersed in a water-based solvent by a water-soluble polymer, via at least one functional group selected from a group consisting of a carboxyl group, an amino group, a diol group, a salicylic acid group, and a phosphoric acid group, and wherein the titanium oxide conjugated particles are further modified with an antibody via the linker molecule. This antitumor agent is concentrated in the affected area and can be utilized as an agent for diagnosis or for treatment in combination with ultrasonic irradiation.

## Description

### BACKGROUND OF THE INVENTION

### Field of the Invention

The present invention relates to an antitumor agent having catalytic activity exhibited upon ultrasonic irradiation, comprising titanium oxide-antibody conjugated particles, wherein a linker molecule is bound to the titanium oxide surface of the titanium oxide conjugated particles dispersed in a water-based solvent by a water-soluble polymer without changing the nature of the water-soluble polymer, and wherein an antibody is further bound via the linker molecule.

### Description of Related Art

Titanium oxide has an isoelectric point at a pH value of around 6. Accordingly, in a nearly neutral water-based solvent, titanium oxide particles form aggregates and it is extremely difficult to disperse the particles homogeneously. Therefore, there have heretofore been made various attempts to disperse titanium oxide particles homogeneously in water-based dispersion media.

It is known that dispersibility of titanium oxide particles in a dispersion medium is improved by addition of PEG (polyethylene glycol) as a dispersant (see Patent Citation 1 (Japanese Patent Laid-Open Publication No. H2-307524) and Patent Citation 2 (Japanese Patent Laid-Open Publication No. 2002-60651)).

Alternatively, fine particles of surface-modified titanium oxide are also known, where hydrophilic polymers such as polyacrylic acid and the like are bound to the fine particles of titanium oxide via carboxyl groups (see Patent Citation 3 (WO 2004/087577)). This technique allows for use of anionic polymers such as polyacrylic acid. Functional groups such as a carboxyl group contained in the anionic polymers provide the fine particles of titanium oxide with a surface charge, whereby the particles exhibit stable dispersibility even in neutral physiological saline, which is close to an in vivo environment, and, also, a function of photocatalytic activity is exhibited upon ultraviolet irradiation.

Further, there are studies being made to provide titanium oxide with functions. For example, with regard to the above-mentioned fine particles of surface-modified titanium oxide, there has been proposed a titanium dioxide conjugate having molecular discrimination ability, wherein a molecule having specific binding ability to a target molecule is fixed to a carboxyl residue not involved in the binding of the hydrophilic polymer mentioned above (see Patent Citation 4 (Japanese Patent No. 3835700)). In this technique, the functional groups such as a carboxyl group contained in the anionic polymer provide a surface charge to the particles even when the molecule is fixed, thus showing stable dispersibility. On the other hand, the surface charge provided by the functional groups directly contributes to dispersibility, while fixing of a molecule to the residue not involved in the binding results in decrease of the surface charge. This puts a limitation on the amount and the like of the molecule to be fixed.

[Patent Citation 1]
   Japanese Patent Laid-Open Publication No. H2-307524
[Patent Citation 2]
   Japanese Patent Laid-Open Publication No. 2002-60651
[Patent Citation 3]
   WO 2004/087577
[Patent Citation 4]
   Japanese Patent No. 3835700

### SUMMARY OF THE INVENTION

It is an object of the present invention to provide an antitumor agent, comprising titanium oxide-antibody conjugated particles provided with specific binding ability without losing dispersibility and catalytic activity, by modifying titanium oxide conjugated particles with an antibody via a linker molecule without changing the nature of a water-soluble polymer, wherein the titanium oxide conjugated particles maintain dispersibility in a water-based solvent by the water-soluble polymer and have catalytic ability exhibited upon ultrasonic irradiation.

The present inventors have recently found that, by binding a linker molecule to the titanium oxide surface of titanium oxide conjugated particles, dispersed in a water-based solvent by a water-soluble polymer, via at least one functional group selected from a group consisting of a carboxyl group, an amino group, a diol group, a salicylic acid group, and a phosphoric acid group, it is possible to newly provide the titanium oxide conjugated particles with an antibody without changing the nature of the water-soluble polymer, while maintaining dispersibility and catalytic activity.

That is, according to the antitumor agent of the present invention, by binding a linker molecule to the titanium oxide surface of titanium oxide conjugated particles dispersed in a water-based solvent by a water-soluble polymer, and further by binding an antibody via the linker molecule, there can be prepared an antitumor agent, comprising titanium oxide-antibody conjugated particles which maintain good dispersibility without changing the nature of the water-soluble polymer, possess catalytic activity exhibited upon ultrasonic irradiation, and are capable of binding with an antigen. By irradiating ultrasonic waves onto the antitumor agent in a state where the agent is bound with the antigen, it is expected that the reactivity between radical species and the antigen is improved. When the antigen is derived from cancer cells or tissues neighboring the cancer such as neovascular tissues or the like, a high antitumor effect can be obtained by concentrating the antitumor agent in the tissue neighboring the cancer, i.e., the affected area, and, further, by carrying out ultrasonic irradiation. Therefore, the antitumor agent of the present invention can be applied as an agent which enhances an ultrasonic cancer treatment conducted by concentrating the agent in the affected area and, further, carrying out ultrasonic irradiation.

According to the present invention, there is provided an antitumor agent comprising titanium oxide-antibody conjugated particles which exhibit catalytic activity upon ultrasonic irradiation, comprising:
titanium oxide conjugated particles comprising titanium oxide particles and a water-soluble polymer bound to the surface of the titanium oxide particles via at least one functional group selected from a group consisting of a carboxyl group, an amino group, a diol group, a salicylic acid group, and a phosphoric acid group;
a linker molecule further bound to the surface of the titanium oxide conjugated particles,
the linker molecule being a compound:
   (1) having at least one functional group selected from a group consisting of a carboxyl group, an amino group, a diol group, a salicylic acid group, and a phosphoric acid group and
   (2) having a) a saturated or unsaturated chain hydrocarbon group having 6 to 40 carbon atoms, b) a substituted or unsubstituted, saturated or unsaturated 5- or 6-membered heterocyclic group, or c) a substituted or unsubstituted, saturated or unsaturated 5- or 6-memebered cyclic hydrocarbon group, and
the linker molecule being bound to the titanium oxide via the functional group without polymerization of the functional groups with each other; and
an antibody being further bound to the titanium oxide via the linker molecule.

According to the present invention, there is also provided a dispersion liquid comprises the antitumor agent and a solvent in which the antitumor agent is dispersed.

According to the present invention, it is possible to provide an antitumor agent and a dispersion thereof, comprising titanium oxide-antibody conjugated particles provided with specific binding ability without losing dispersibility and catalytic activity, by modifying titanium oxide conjugated particles with an antibody via a linker molecule without changing the nature of a water-soluble polymer, wherein the titanium oxide conjugated particles maintain dispersibility in the water-based solvent by the water-soluble polymer and have catalytic ability exhibited upon ultrasonic irradiation.

### BRIEF DESCRIPTION OF THE DRAWINGS

Fig. 1 is a diagram showing an example of an antitumor agent of the present invention.
Fig. 2 is a diagram showing fluorescence intensity via a fluorescent reagent for detection of singlet oxygen, measured for various particles in Example 9, where the fluorescence is due to generation of singlet oxygen by ultrasonic irradiation.
Fig. 3 shows evaluation results of binding of titanium oxide-antibody conjugated particles to an antigen, measured in Example 10.
Fig. 4 is a graph showing fluorescent intensity via a fluorescent reagent for detection of hydroxyl radicals, measured for titanium oxide conjugated particles E in Example 15, where the fluorescence is due to generation of hydroxyl radicals after ultrasonic irradiation.

### DETAILED DESCRIPTION OF THE INVENTION

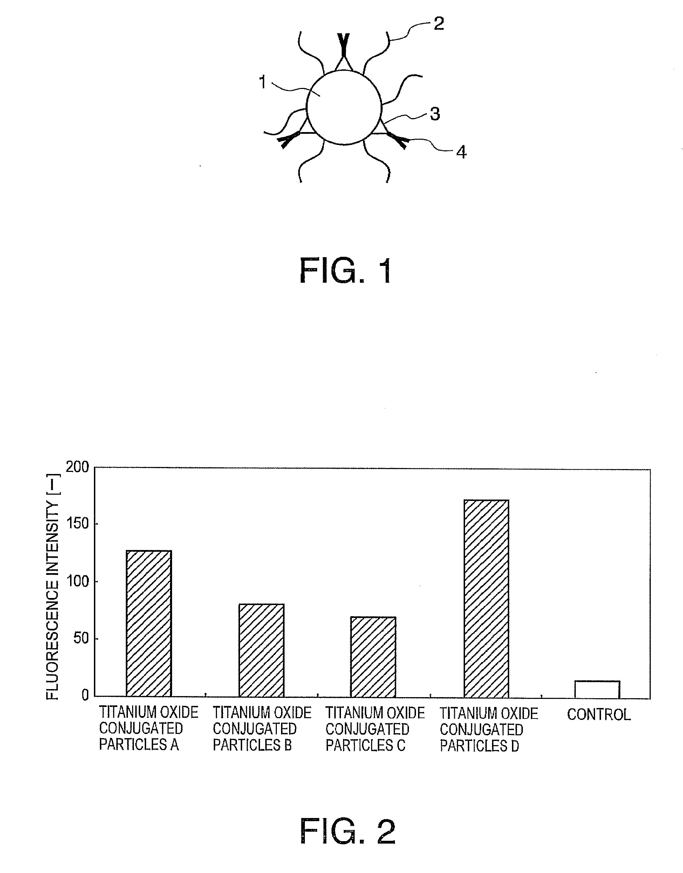

The antitumor agent according to the present invention comprises titanium oxide-antibody conjugated particles, containing a titanium oxide particle, a water-soluble polymer, a linker molecule, and an antibody. In Fig. 1 is shown an example of the antitumor agent. As is shown in Fig. 1, the antitumor agent comprises a titanium oxide particle 1, to which surface are bound a water-soluble polymer 2 and an antibody 4 via a linker molecule 3. The bonds between the titanium oxide particle 1 and the water-soluble polymer 2 and the linker molecule 3 are formed via at least one functional group selected from a carboxyl group, an amino group, a diol group, a salicylic acid group, and a phosphoric acid group.

More specifically, because these functional groups form a strong bond with titanium oxide, dispersibility can be retained despite high catalytic activity of the titanium oxide particles. Also, it is possible to maintain binding of the antibody via the linker molecule. In addition, the bonding form in the present invention may be such that dispersibility is secured 24 to 72 hours after administration into the body, from a viewpoint of safety in the body. The bonding form is preferably a covalent bond because it provides stable dispersion under physiological conditions, does not cause isolation of the water-soluble polymer even after ultrasonic irradiation, and does little damage to normal cells.

The carboxyl group, amino group, diol group, salicylic acid group, and phosphoric acid group do not polymerize with each other unlike functional groups such as a trifunctional silanol group which undergoes condensation-polymerization with each other three-dimensionally to cover the entire surface of the titanium oxide particle with the resultant polymer. Thus, it is thought that, in the case of these groups, bare portions are secured on the surface of the titanium oxide particle as shown in Fig. 1. As a result, the catalytic activity of the titanium oxide particles can be fully exhibited, while suppressing deactivation thereof which may be caused by covering of the entire surface with the polymer.

Further, the water-soluble polymer bound to the surface of the titanium oxide particles can disperse the antitumor agent of the present invention by an effect of electric charge or hydration in a nearly neutral water-based solvent, wherein dispersion of titanium oxide particles is thought to be difficult. A method to introduce an antibody to a water-soluble polymer, which is bound to the surface of titanium oxide particles, is publicly known. In this case, it is necessary that the water-soluble polymer contains a polar group with high reactivity in order to form a chemical bond between the water-soluble polymer and the antibody. This polar group contained in the water-soluble polymer is lost upon binding of the antibody. Because of this, a change occurs in the polarity itself of the water-soluble polymer. Namely, it is thought that the balance, maintained in dispersion of the titanium oxide particles by the effect of electric charge or hydration of the water-soluble polymer bound to the surface of the titanium oxide particles, changes before and after binding of the antibody. Maintenance of the dispersed state can be accomplished only by controlling well the balance of the electric charge or hydration accompanying this change in the nature of the water-soluble polymer bound to the surface of the titanium oxide particles. On the other hand, regarding the antibody bound via a linker molecule which is bonded to the surface of the titanium oxide particles in the present invention, high dispersibility due to the water-soluble polymer can be maintained by binding the antibody without changing the nature of the water-soluble polymer. Thus, it is possible to design molecules with high degree of freedom in binding the antibody, without giving consideration to a change in dispersibility which may be caused by a change in the nature of the water-soluble polymer.

According to the antitumor agent of the present invention, by binding a linker molecule to the titanium oxide surface of the titanium oxide conjugated particles dispersed in a water-based solvent by a water-soluble polymer, and further by binding an antibody via the linker molecule, there can be prepared an antitumor agent, comprising titanium oxide-antibody conjugated particles which maintain high dispersibility without changing the nature of the water-soluble polymer. By thus binding the antibody, it becomes possible for the antitumor agent of the present invention to bind with an antigen. Also, ultrasonic irradiation on the antitumor agent of the present invention can produce radical species. In general, the radical species have high reactivity but have a short lifetime, and react with neighboring materials after diffusing only a short distance. Thus, by carrying out ultrasonic irradiation in a state in which the antitumor agent is bound with the antigen, it can be expected that the reactivity between the radical species and the antigen is improved. When the antigen is derived from the cancer cells or tissues neighboring the cancer such as neovascular tissues and the like, a high antitumor effect can be obtained by concentrating the antitumor agent of the present invention in the affected area, namely in the proximity of the cancer, and further by carrying out ultrasonic irradiation. Therefore, the antitumor agent of the present invention can be expected to exhibit an effect as an agent to enhance the ultrasonic cancer treatment, which is carried out by concentrating the antitumor agent in the affected area after administration thereof, and further irradiating the area with ultrasonic waves.

Also, according to the antitumor agent of the present invention, by binding a photosensitive molecule or a radioactive material to the surface of the titanium oxide-antibody conjugated particles via the linker molecule, high dispersibility can be maintained without changing the nature of the water-soluble polymer. Especially, with regard to the radioactive material, it is necessary to use as few steps as possible from a safety viewpoint. The particles can be labeled by a few simple steps, in which unbound radioactive material is removed by separation using an appropriate method, after the radioactive material is bonded to the titanium oxide surface of the titanium oxide conjugated particles dispersed in a water-based solvent by the water-soluble polymer. Therefore, there is little chance for the radioactive material to spread outside and this preparative method is superior in terms of safety. Also, by measuring these particles by an appropriate instrument, it is possible to carry out imaging and quantitative measurement of the particles. Thus, the antitumor agent of the present invention can also be utilized as a material for a tracer experiment to confirm the dynamic state of the agent after administration in the body and as a medical material for diagnosis and treatment conducted by ultrasonic irradiation on the affected area.

In a preferable embodiment of the present invention, the water-soluble polymer used in the present invention is preferably bound to the surface of the titanium oxide particles via at least one functional group selected from a group consisting of a carboxyl group, an amino group, a diol group, a salicylic acid group, and a phosphoric acid group. This enables the polymer to bind to the surface of titanium oxide strongly. Also, because the functional groups do not polymerize with each other, unlike functional groups such as a trifunctional silanol group which undergoes condensation-polymerization with each other three-dimensionally to cover the entire surface of the titanium oxide particles with the resultant polymer, it is thought that a considerable amount of bare portions are secured on the surface of the titanium oxide particles as shown in Fig. 1. As a result, the catalytic activity of the titanium oxide particles can be fully exhibited, while suppressing deactivation caused by covering of the entire surface thereof with the polymer.

In a preferable embodiment of the present invention, the water-soluble polymer used in the present invention is not particularly limited as long as it can disperse the titanium oxide-antibody conjugated particles in a water-based solvent. In the water-soluble polymer used in the present invention, one having an electric charge includes a water-soluble polymer with cationicity or anionicity, and one which has no electric charge and provides dispersibility via hydration includes a water-soluble polymer with nonionicity; the water-soluble polymer used in the present invention contains at least one kind of these.

In a preferable embodiment of the present invention, the water-soluble polymer has a weight average molecular weight of 2000 to 100000. The weight average molecular weight of the water-soluble polymer is a value obtained by size exclusion chromatography. With the molecular weight being in this range, the titanium oxide-antibody conjugated particles can be dispersed in a nearly neutral water-based solvent by the effect of the electric charge or hydration due to the water-soluble polymer, wherein dispersion of titanium oxide particles is said to be difficult. The more preferable range is 5000 to 100000, further preferably 5000 to 40000.

In a preferable embodiment of the present invention, any water-soluble polymer with anionicity can be put to use as the water-soluble polymer used in the present invention, as long as it can disperse the antitumor agent of the present invention in the water-based solvent. As the water-soluble polymer with anionicity, those having a plurality of carboxyl groups can be used preferably, including, for example, carboxymethyl starch, carboxymethyl dextran, carboxymethyl cellulose, polycarboxylic acids, and copolymers having carboxyl group units. Specifically, from a viewpoint of hydrolysis and solubility of the water-soluble polymer, more preferably used are polycarboxylic acids such as polyacrylic acid, polymaleic acid, and the like, and copolymers of acrylic acid/maleic acid or acrylic acid/sulfonic-acid type monomer, further preferably polyacrylic acid.

When polyacrylic acid is used as the water-soluble polymer with anionicity, the weight average molecular weight of the polyacrylic acid is, from a viewpoint of dispersibility, preferably 2000 to 100000, more referably 5000 to 40000, further preferably 5000 to 20000.

In a preferable embodiment of the present invention, any water-soluble polymer with cationicity can be put to use as the water-soluble polymer used in the present invention, as long as it can disperse the antitumor agent of the present invention in the water-based solvent. As the water-soluble polymer with cationicity, those having a plurality of amino groups can be used preferably, including, for example, polyamino acid, polypeptide, polyamines, and copolymers having amine units. Specifically, from a viewpoint of hydrolysis and solubility of the water-soluble polymer, more preferably used are polyamines such as polyethyleneimine, polyvinylamine, polyallylamine, and the like, further preferably polyethyleneimine.

When polyethyleneimine is used as the water-soluble polymer with cationicity, the weight average molecular weight of the polyethyleneimine is, from a viewpoint of dispersibility, preferably 2000 to 100000, more referably 5000 to 40000, further preferably 5000 to 20000.

In a preferable embodiment of the present invention, any water-soluble polymer with nonionicity can be put to use as the water-soluble polymer used in the present invention, as long as it can disperse the antitumor agent of the present invention in the water-based solvent. As the water-soluble polymer with nonionicity, those having hydroxyl groups and/or polyoxyalkylene groups can preferably be mentioned. Preferable examples of such water-soluble polymers include polyethylene glycol (PEG), polyvinyl alcohol, polyethylene oxide, dextran, or copolymers having these, more preferably polyethylene glycol (PEG) and dextran, further preferably polyethylene glycol.

When polyethylene glycol is used as the water-soluble polymer with nonionicity, the weight average molecular weight of the polyethylene glycol is, from a viewpoint of dispersibility, preferably 2000 to 100000, more referably 5000 to 40000.

The water-soluble polymers exemplified above may be used freely in combination with each component described heretofore, in so far as dispersibility of the antitumor agent of the present invention is not lost.

In a preferable embodiment of the present invention, the linker molecule used in the present invention is bound to the surface of the titanium oxide particles and the functional molecule has at least one functional group selected from a group consisting of a carboxyl group, an amino group, a diol group, a salicylic acid group, and a phosphoric acid group.

In a preferable embodiment of the present invention, the linker molecule used in the present invention is a compound containing a) a saturated or an unsaturated chain hydrocarbon group having 6 to 40 carbons, b) a substituted or unsubstituted, saturated or unsaturated 5- or 6-membered heterocyclic group, or c) a substituted or unsubstituted, saturated or unsaturated 5- or 6-memebered cyclic hydrocarbon group.

The linker molecule having the above-described number of carbons has a smaller molecular weight than the aforementioned water-soluble polymer. Also, the linker molecule is bound to the surface of titanium dioxide. Therefore, the titanium oxide-antibody conjugated particle of the present invention takes a structure in which the linker molecule is contained in an inner position while the water-soluble polymer is situated in the outer shell. The outer shell has the larger effect on dispersibility of the antitumor agent of the present invention. Namely, compared to the water-soluble polymer situated in the outer shell, the linker molecule positioned inside has a smaller effect on the dispersibility and can be used preferably.

The amount of the linker molecule bound to the antitumor agent of the present invention is 1.0 × 10⁻⁶ to 1.0 × 10⁻³ mol per 1 g mass of the titanium oxide particles, more preferably 1.0 × 10⁻⁶ to 1.0 × 10⁻⁴ mol/g-titanium oxide particle. Within the range, the antitumor agent of the present invention can be used preferably because it can be dispersed in a 10% protein solution used as a solvent that is close to the in vivo environment. Further, within the range, the antitumor agent of the present invention can be used preferably because it can exhibit catalytic activity upon ultrasonic irradiation by generating radical species.

As examples of such linker molecules, there may be envisioned aromatic compounds, molecules having alkyl structures, and the like. More specifically, the molecules having a benzene ring include catechols having a catechol structure in the molecule such as catechol, methyl catechol , tert-butylcatechol, dopamine, dihydroxyphenylethanol, dihydroxyphenylpropionic acid, dihydroxyphenylacetic acid, and the like. Also, as other cyclic molecules, there may preferably be used ferrocene, ferrocenecarboxylic acid, ascorbic acid, dihydroxycyclobutenediene, alizarin, binaphthalenediol, and the like. Further, the molecules having an alkyl structure include molecules containing alkyl groups such as a hexyl group, an octyl group, a lauryl group, a palmityl group, a stearyl group, and the like. Alternatively, there may be mentioned molecules having saturated or unsaturated aliphatic hydrocarbon groups including alkenyl groups such as a hexenyl group, an octenyl group, an oleyl group, and the like, and a cycloalkyl group.

The linker molecules and the amount thereof exemplified above may be combined suitably with each constituent component described heretofore.

In a preferable embodiment of the present invention, the antibody bound via the linker molecule is not particularly limited but, in order to actively concentrate the antitumor agent of the present invention in the cancerous region, an antigen for the antibody is preferably derived from the cancer cells or tissues neighboring the cancer such as neovascular tissues or the like. Alternatively, there is no problem to use fragments obtained by cleaving the antibody into the Fab region and the like.

In addition, in order to actively concentrate the antitumor agent of the present invention in the cancerous region, the molecule which binds via the linker molecule is not limited to the antibody but may include peptides and amino acid sequences which, for example, show mutual interactions with the cancer cells or regions neighboring the cancer such as neovascular tissues or the like. More specifically, there may be mentioned 5-aminolevulinic acid, methionine, cysteine, glycine, and the like. Alternatively, the molecule may contain a sugar chain. Further, the molecule may contain a nucleic acid having binding ability. The nucleic acid is not particularly limited and there may be used nucleic-acid bases such as DNA, RNA, and the like; peptide nucleic acids such as PNA and the like; or aptamers, which are the higher order structures formed by these, and the like. The above-described peptides and amino acid sequences may be used in combination with the antibody. Also, these peptides and amino acid sequences may be used suitably in combination with each constitutional component described heretofore.

In a preferable embodiment of the present invention, there may be bound different functional molecules via the linker molecule other than the antibody which is bound via the linker molecule. Examples of the functional molecules include a photosensitive molecule and, as the photosensitive molecule, there may be used a fluorescent molecule.

Also, other examples of the functional molecules include a radioactive compound. The radioactive compound includes a compound containing an isotope element. For example, ¹⁴C-labeled catechol and the like having ¹⁴C may be used suitably.

Further, other examples of the functional molecules include a radical-responsive compound. The radical-responsive compound includes a chemiluminescent molecule and a fluorescent molecule, which show specific reactivity with radicals, or a spin trapping agent. More specifically, the chemiluminescent molecules and fluorescent molecules include luminol, sea firefly luciferin analogues, oxalic acid ester, acridinium, para-hydroxyphenyl fluorescein, para-aminophenyl fluorescein, dihydrorhodamine 123, dihydrorhodamine 6G, trans-1-(2'-methoxyvinyl)pyrene, dihydroxyethidium, folic acid, (2',7'-dichlorodihydrofluorescein diacetate, succinimidyl ester) (Invitrogen Corporation), 5- or 6-(N-succinimidyloxycarbonyl)-3',6'-O,O'-diacetylfluoroscein, Cy dye (manufactured by Amersham Biosciences), pterin, and the like. Spin trapping agents include 4,6-tri-tert-butylnitrosobenzene, 2-methyl-2-nitrosopropane, 3,3,5,5-tetramethyl-1-pyrroline N-oxide, 5,5-dimethyl-1-pyrroline N-oxide, 5-(diethylphosphono)-5-methyl-1-pyrroline N-oxide, N-tert-butyl-alpha-(4-pyridyl-1-oxide)nitrone, N-tert-butyl-alpha-phenylnitrone, nitrosobenzene, 5,5-dimethyl-1-pyrroline N-oxide, 4-hydroxy-2,2,6,6-tetramethylpiperidinyloxy free radical, 2-(5,5-dimethyl-2-oxo-2λ5-[1,3,2]dioxaphosphinan-2-yl)-2-methy l-3,4-dihydro-2H-pyrrole 1-oxide, 5-diethoxyphosphoryl-5-methyl-1-pyrroline N-oxide, and the like.

Further, other examples of the functional molecules include at least one anticancer agent selected from fluorouracil, gemcitabine, methotrexate, cyclophosphamide, daunorubicin hydrochloride, adriamycin, idarubicin hydrochloride, bleomycin, mitomycin, actinomycin, vincristine, cisplatin, carboplatin, etoposide, nedaplatin, paclitaxel, docetaxel, irinotecan hydrochloride, and the like; antibacterial agents such as penicillin types, macrolide types, new quinolone types, tetracycline types, and the like; antiviral drugs such as lamivudine, nelfinavir, indinavir, saquinavir, interferon, amantadine, aciclovir, and the like; hormonal disorder drugs such as leuprorelin, buserelin, goserelin, triptorelin, nafarelin, and the like; analgetic drugs such as ibuprofen and the like.

Also, other examples of the functional molecules include a molecule containing a low-valent transition metal. The low-valent transition metal is known to decompose hydrogen peroxide by the Harber-Weiss mechanism to produce hydroxyl radicals (Chemistry of Active Oxygen Species [Quarterly Chemical Review No. 7], edited by Japan Chemical Society) and when, as the low-valent transition metal, for example, a divalent iron ion is used, the reaction is well known as the Fenton reaction. In addition, various radicals including the hydroxyl radical are known to possess a cytopathic effect. Therefore, if a molecule containing these low-valent transition metals is bound via a linker molecule, radicals can be generated and the cytopathic effect can be maintained as long as hydrogen peroxide is present. Namely, even after ultrasonic irradiation is stopped, hydroxyl radicals having stronger oxidative ability is continuously generated by the Fenton reaction between hydrogen peroxide built up in the system and the molecule containing the low-valent transition metal bound to the antitumor agent of the present invention. And, accompanying this, it is possible to obtain a lasting antitumor effect. However, when a complex is used as the molecule containing the low-valent transition metal, it is thought that not only free hydroxyl radicals but also, for example, a ferryl complex and the like, which may be generated when an iron complex is used, may get involved in an oxidation reaction in the form of so-called Crypto-HO·. These low-valent transition metals include, in addition to the divalent iron, trivalent titanium, divalent chromium, monovalent copper, and the like. Further, the molecules containing these low-valent transition metals include ferrocene carboxylic acid, a complex between bicinchoninic acid and monovalent copper, and the like.

The functional molecules mentioned above may be suitably used in combination with each constituent unit described heretofore and may achieve the above-described various effects without hindering the effect of the present invention.

In a preferable embodiment of the present invention, there is no problem even if the linker molecule used in the present invention is a molecule in which the above-described functional molecule and the functional group bound to the titanium oxide surface are bound via another linker.

In a preferable embodiment of the present invention, as the aforementioned linker, there may be conceived a heterobifunctional crosslinker used when, for example, biomolecules are bound to each other by different functional groups. Specific examples of the crosslinkers include N-hydroxysuccinimide, N-[α-maleimidoacetoxy]succinimide ester, N-[β-maleimidopropyloxy]succinimide ester, N-β-maleimidopropionic acid, N-[β-maleimidopropionic acid] hydrazide/TFA, 1-ethyl-3-[3-dimethylaminopropyl]carbodiimide hydrochloride, N-ε-maleimidocaproic acid, N-[ε-maleimidocaproic acid]hydrazide, N-[ε-maleimidocaproyloxy]succinimide ester, N-[γ-maleimidobutyryloxy]succinimide ester, N-κ-maleimidoundecanoic acid, N-[κ-maleimidoundecanoic acid]hydrazide, succinimidyl 4-[N-maleimidomethyl]-cyclohexane-1-carboxy-[6-amidocaproate], succinimidyl 6-[3-(2-pyridyldithio)-propionamide]hexanoate, m-maleimidobenzoyl-N-hydroxysuccinimide ester, 4-[4-N-maleimidophenyl]butyric acid hydrazide/HCl, 3-[2-pyridyldithio]propionylhydrazide, N-[p-maleimidophenyl] isocyanate , N-succinimidyl[4-azidophenyl]-1,3'-dithiopropionate, N-succinimidyl S-acetylthioacetate, N-succinimidyl S-acetylthiopropionate, succinimidyl 3-[bromoacetamido]propionate, N-succinimidyl iodoacetate, N-succinimidyl[4-iodoacetyl]aminobenzoate, succinimidyl 4-[N-maleimidomethyl]-cyclohexane-1-carboxylate, succinimidyl 4-[p-maleimidophenyl]butyrate, succinimidyl 6-[(β-maleimidopropionamide)hexanoate], 4-succinimidyloxycarbonylmethyl-α[2-pyridyldithio]toluene, N-succinimidyl 3-[2-pyridyldithio]propionate, N-[ε-maleimidocaproyloxy]sulfosuccinimide ester, N-[γ-maleimidobutyryloxy]sulfosuccinimide ester, N-[κ-maleimidoundecanoyloxy]sulfosuccinimide ester, sulfosuccinimidyl-6-[α-methyl-α-(2-pyridyldithio)toluamide] hexanonate, sulfosuccinimidyl 6-[3'-(2-pyridyldithio)propionamide]hexanoate, m-maleimidobenzoyl-N-hydroxysulfosuccinimide ester, sulfosuccinimidyl [4-iodoacetyl]aminobenzoate, sulfosuccinimidyl 4-[N-maleimidomethyl]cyclohexane-1-carboxylate, sulfosuccinimidyl 4-[p-maleimidophenyl]butyrate, N-[ε-trifluoroacetylcaproyloxy]succinimide ester, chlorotriazine, dichlorotriazine, trichlorotriazine, succinimidyl-4-hydrazino nicotinate-acetone hydrazone, C6-succinimidyl-4-hydrazino nicotinate-acetone hydrazone, succinimidyl-4-hydrazido terephthalate hydrochloride, succinimidyl-4-formylbenzoate, C6-succinimidyl-4-formylbenzoate, and the like. Further, the linker may be composed of a plurality of kinds of linkers, to which other linkers may be bound. The above-mentioned linkers may be used suitably in combination with each constituent unit described heretofore.

In a preferable embodiment of the present invention, the diol group used for binding the titanium oxide particles with a water-soluble polymer and/or a linker molecule is preferably an enediol group, more preferably an α-diol group. By using these functional groups, excellent binding to the titanium oxide particles can be realized.

In a preferable embodiment of the present invention, the titanium oxide particles are preferably anatase-type titanium oxide or rutile-type titanium oxide. When high catalytic activity by irradiation with ultraviolet light or ultrasonic waves is utilized, anatase-type titanium oxide is preferable, and when properties such as a high refractive index and the like are utilized as in cosmetic products, rutile-type titanium oxide is preferable. The use of anatase-type titanium oxide or rutile-type titanium oxide as the titanium oxide particles may be combined suitably with each constituent unit described heretofore and makes it possible to achieve the above-mentioned new effect.

In a preferable embodiment of the present invention, the antitumor agent of the present invention has a particle diameter of 20 to 200 nm, more preferably 50 to 200 nm, further preferably 50 to 150 nm. Within this range of particle diameter, when the antitumor agent is administered into the patient's body with an aim for the agent to reach the cancerous tumor, as in a drug delivery system, the agent efficiently reaches the cancer tissue and is concentrated therein by Enhanced Permeability and Retention Effect (EPR effect). And, as described above, upon irradiation with ultrasonic waves of 400 kHz to 20 MHz, specific generation of radical species takes place. Accordingly, the cancer tissue can be killed with high efficiency by ultrasonic irradiation.

In another preferable embodiment of the present invention, when the antitumor agent has a particle diameter of less than 50 nm (for example, several nm), the EPR effect can also be obtained by increasing the apparent size. Namely, by linking semiconductor particles with each other, for example, by a method of binding with a polyfunctional linker and the like, so as to form a secondary particle having a particle diameter of 50 to 150 nm, a high cancer treatment effect can be realized by the EPR effect.

In the present invention, the particle diameter of the semiconductor particles can be measured by a dynamic light scattering method. Specifically, the particle diameter can be obtained as a value expressed in terms of the Z-average size, obtained by a cumulant analysis using a particle size distribution measuring apparatus (Zetasizer Nano, manufactured by Malvern Instruments Ltd.).

Adjustment of the particle diameter of the antitumor agent of the present invention in the aforementioned range makes it possible to combine suitably with each constituent unit described heretofore and to achieve the above-mentioned new effect.

The antitumor agent of the present invention includes not only a single kind of titanium oxide conjugated particles but also a mixture of a plurality of kinds of semiconductor particles or compounds thereof. Specific examples include a compound of titanium oxide particles and iron oxide nano-particles, a compound of titanium oxide particles and platinum, silica-coated titanium oxide, and the like.

In a preferable embodiment of the present invention, the antitumor agent of the present invention is preferably dispersed in a solvent and is in a form of a dispersion liquid. By virtue of this, the antitumor agent of the present invention can be used as an antitumor agent which can be efficiently administered into the patient's body by various methods such as instillation, injection, coating, and the like. The liquid property of the dispersion liquid is not limited, and a high dispersibility can be realized over a wide pH range of 3 to 10. In addition, from a viewpoint of safety in administration into the body, the dispersion liquid preferably has a pH of 5 to 9, more preferably 5 to 8. Especially, one with a neutral liquid property is prefrable. Also, in a preferable embodiment of the present invention, the solvent is preferably a water-based solvent, more preferably a pH buffer solution or physiological saline. The preferable salt concentration of the water-based solvent is 2 M or less, more preferably 200 mM or less from a safety viewpoint of administration into the body. The antitumor agent of the present invention is contained in the dispersion liquid preferably in an amount of 0.001 to 1% by mass, more preferably 0.001 to 0.1% by mass. Within this range, the particles can be effectively concentrated in the affected area (tumor) 24 to 72 hours after the administration. Namely, it becomes easier for the particles to be concentrated in the affected area (tumor) and, at the same time, there is no fear of inviting a secondary negative effect such as obstruction of blood vessels after the administration because dispersibility of the particles in the blood can be ensured and, consequently, aggregates are less likely to be formed.

The antitumor agent of the present invention can be administered into the patient's body by various methods such as instillation, injection, coating, and the like. Especially, the use thereof via an intravenous or subcutaneous administration route is preferable from a viewpoint of reducing patient burden, by a so-called DDS-like treatment utilizing the EPR effect due to the particle size, retention in blood, and mutual interaction between the antibody bound to the particle and an antigen derived from the affected area. The titanium oxide-antibody conjugated particles administered into the body reach the cancer tissues and get concentrated therein as in a drug delivery system.

The antitumor agent of the present invention, when used by administration routes via blood vessels, body organs, and the like, which are close to the affected area, is preferable from a viewpoint of reducing patient burden by utilizing high dispersibility in an in vivo environment and mutual interaction between the antibody bound to the particle and an antigen derived from the affected area, namely, by virtue of the so-called local DDS-like treatment. Further, the titanium oxide-antibody conjugated particles administered into the body reach the cancer tissue and concentrated therein as in a drug delivery sysem.

The antitumor agent of the present invention can be converted to a cytotoxin upon irradiation with ultrasonic waves or ultraviolet light, preferably ultrasonic waves. This antitumor agent can kill cells by being administered into the body, being subjected to ultrasonic irradiation, and producing a cytotoxin upon the irradiation. It can kill the target cells not only in vivo but also in vitro. In the present invention, the target cells are not particularly limited, but they are preferably the cancer cells. Namely, the antitumor agent of the present invention can be used as a drug to kill the cancer cells upon activation by ultrasonic or ultraviolet irradiation.

In a preferable embodiment of the present invention, ultrasonic treatment is carried out on the cancer tissue wherein the antitumor agent of the present invention has been concentrated. The frequency of the ultrasonic waves used is preferably 400 kHz to 20 MHz, more preferably 600 kHz to 10 MHz, further preferably 1 MHz to 10 MHz. The ultrasonic irradiation time should be properly determined by considering the position and size of the cancer tissue, which is the object of the treatment, and is not particularly limited. In this way, the cancer tissue in the patient can be killed by ultrasonic irradiation with high efficiency to realize a high cancer treatment effect. It is possible to make the ultrasonic waves reach a deep part in the living body from outside and, by using ultrasonic waves in combination with the titanium oxide-antibody conjugated particles of the present invention, treatment of the affected area or the target region, present in a deep part of the living body, can be realized in a noninvasive state. Further, because the antitumor agent of the present invention is concentrated in the affected area or the target region, ultrasonic waves of low intensity, which do not adversely affect the neighboring normal cells, can be made to act only on the local area where the titanium oxide-antibody conjugated particles are concentrated.

It is noted that the effect of these semiconductor particles to kill cells via activation by ultrasonic irradiation can be obtained by generation of radical species upon ultrasonic irradiation. Namely, the biological cell-killing effect provided by the semiconductor particles is considered to be due to a qualitative and quantitative increase in radical species and these radical species are thought to act as a cytotoxin. The reason for this is inferred as follows. However, the following reason is hypothetical and the present invention is not limited in any way by the following description. That is, even though hydrogen peroxide and hydroxyl radicals are generated in the system, the present inventors have found that the generation of hydrogen peroxide and hydroxyl radicals are accelerated in the presence of semiconductor particles such as titanium oxide and the like. Further, in the presence of these semiconductor particles, especially in the presence of titanium oxide, it appears that generation of superoxide anions and singlet oxygen is accelerated. The specific generation of these radical species, when fine particles of nanometer order are used, is thought to be a phenomenon observed substantially when the frequency during ultrasonic irradiation is in a range of 400 kHz to 20 MHz, preferably in a range of 600 kHz to 10 MHz, more preferably in a range of 1 MHz to 10 MHz.

### (Production method)

The titanium oxide conjugated particles of the present invention can be produced by binding a water-soluble polymer to titanium oxide particles, wherein the water-soluble polymer having at least one functional group selected from a carboxyl group, an amino group, a diol group, a salicylic acid group, and a phosphoric acid group. The production of the titanium oxide conjugated particles by this method can be carried out, for example, by dispersing the titanium oxide particles and a nonionic water-soluble polymer having at least one functional group selected from a carboxyl group, an amino group, a diol group, a salicylic acid group, and a phosphoric acid group, in an aprotic solvent and heating the resultant dispersion liquid at 80 to 220°C, for example, for 1 to 16 hours. In addition, preferable examples of the aprotic solvents include dimethylformamide, dioxane, and dimethylsulfoxide.

The antitumor agent of the present invention can be produced by bonding a linker molecule via at least one functional group, selected from a group consisting of a carboxyl group, an amino group, a diol group, a salicylic acid group, and a phosphoric acid group, to the titanium oxide surface of the titanium oxide conjugated particles dispersed in a water-based solvent by a water-soluble polymer and, further, by forming titanium oxide-antibody conjugated particles modified with an antibody via the linker molecule. The production of the antitumor agent by this method can be carried out, for example, by dispersing titanium oxide conjugated particles and a linker molecule having at least one functional group, selected from a carboxyl group, an amino group, a diol group, a salicylic acid group, and a phosphoric acid group, in an aqueous solution; heating, for example, at 0°C to 50°C for 1 to 16 hours; then, after removing the unbound linker molecule by a membrane separation technique and the like, the functional group such as an amino group and the like possessed by the linker molecule which is bound to the titanium oxide conjugated particle, is activated by reaction with carbodiimide reagents such as, for example, 1-ethyl-3-(3-dimethylaminopropyl)carbodiimide hydrochloride and the like; thereafter, the unreacted carbodiimide reagent is removed by a membrane separation technique and the like, and an antibody is mixed and reacted, for example, at 0°C to room temperature for 1 to 16 hours, followed by removal of the unreacted antibody by a membrane separation technique and the like.

Alternatively, the antitumor agent of the present invention can be produced by the following procedure: after a linker molecule is activated by reaction with a carbodiimide reagent such as, for example, 1-ethyl-3-(3-dimethylaminopropyl)carbodiimide hydrochloride and the like, it is reacted with the antibody via at least one functional group, selected from a group consisting of a carboxyl group, an amino group, a diol group, a salicylic acid group, and a phosphoric acid group, for example, at 0°C to room temperature for 1 to 16 hours, and, after removing the unreacted linker molecule by a membrane separation technique and the like, titanium oxide particles dispersed in a water-based solvent by a water-soluble polymer are mixed therein and reacted, for example, at 0°C to room temperature for 1 to 16 hours to bind the conjugate between the antibody and the linker molecule to the titanium oxide surface, followed by removal of the unreacted antibody by a membrane separation technique and the like.

### EXAM PLES

In the following, examples are shown. Unless otherwise noted, "%" refers to % by mass.

### Example 1: Preparation of polyethylene glycol-bound titanium oxide conjugated particles

Titanium tetra-isopropoxide (3.6 g) was mixed with 3.6 g of isopropanol and hydrolysis was carried out by adding the resultant mixture dropwise to 60 ml of ultrapure water under ice cooling. After the dropwise addition was complete, the mixture was stirred at room temperature for 30 minutes. After stirring, 1 ml of 12 N nitric acid was added dropwise thereto and the mixture was stirred at 80°C for 8 hr to achieve peptization. After completion of the peptization, the reaction mixture was filtered through a 0.45 µm filter and was further subjected to a solution exchange using a desalting column, PD-10 (manufactured by GE Healthcare Bioscience), to prepare an acidic titanium oxide sol having a solid content of 1 %. This titanium oxide sol was placed in a 100 ml vial bottle and was subjected to ultrasonic treatment at 200 kHz for 30 minutes using an ultrasonic generator MIDSONIC 200 (manufactured by Kaijo Corp.). The average dispersed particle diameter after the ultrasonic treatment was measured by a dynamic light scattering method. This measurement was carried out at 25°C, after diluting the titanium oxide sol treated with ultrasonic waves with 12 N nitric acid by a factor of 1000, by charging 0.1 ml of the dispersion liquid in a quartz measurement cell, using Zetasizer Nano ZS (manufactured by Sysmex Corporation), and setting various parameters of the solvent to the same values as those for water. As a result, the dispersed particle diameter was found to be 36.4 nm. Using an evaporating dish, the titanium oxide sol solution was concentrated at 50°C and, finally, an acidic titanium oxide sol with a solid content of 20% was prepared.

Then, a solution obtained by hydrolyzing 1 g of a copolymer of polyoxyethylene-monoallyl-monomethyl ether and maleic anhydride (average molecular weight; 33659, manufactured by NOF Co., Ltd.) by adding 5 ml of water and 1-ethyl-3-(3-dimethylaminopropyl)carbodiimide hydrochloride (manufactured by Dojindo Laboratories) were mixed and adjusted with ultrapure water so that their respective concentrations became 50 mg/ml and 50 mM. To the adjusted solution was mixed 4-aminosalicilic acid (molecular weight, Mn = 153.14; manufactured by MP Biomedicals, Inc.) so that its concentration became 50 mM and thus a 4 ml solution was obtained. This solution was stirred by shaking to react at room temperature for 72 hours. After completion of the reaction, the solution obtained was transferred to Spectra/Pore CE dialysis tubing (cut-off molecular weight = 3500; Spectrum Laboratories, Inc.), a dialytic membrane, and was dialyzed against 4 l of ultrapure water at room temperature for 24 hours. After dialysis was complete, the whole solution was transferred to an eggplant-shaped flask and freeze dried overnight. To the powder obtained was added 4 ml of dimethylformamide (DMF: manufactured by Wako Pure Chemical Ind., Ltd.) and mixed to provide a solution of 4-aminosalicylic acid-bound polyethylene glycol.

Then, the solution of 4-aminosalicylic acid-bound polyethylene glycol and the anatase-type titanium dioxide sol obtained previously were mixed and the concentrations were adjusted by using DMF so that the final concentration of the former became 20% (vol/vol) and the final solid content of the latter became 0.25%, to provide a 2.5 ml reaction solution. This reaction solution was transferred to a hydrothermal reaction vessel, HU-50 (manufactured by San-Ai Science Co., Ltd.), and was reacted for 6 hours under heating at 80°C. After completion of the reaction, the reaction mixture was cooled until the vessel temperature became 50°C or lower, DMF was removed by an evaporator, and subsequently 1 ml of distilled water was added to obtain a dispersion liquid of polyethylene glycol-bound titanium oxide conjugated particles. Further, when the dispersion liquid was subjected to HPLC [AKTA Purifier (manufactured by GE Healthcare Bioscience), column: HiPrep 16/60 Sephacryl S-300HR (manufactured by GE Healthcare Bioscience), mobile phase: phosphate buffer solution (pH 7.4), flow rate: 0.3 ml/min], a UV absorption peak was observed in a fraction which passed through the column, and this fraction was recovered. This dispersion liquid was diluted with distilled water to a 0.05% (wt/vol) aqueous solution and allowed to stand still for 72 hours. Thereafter, the dispersed particle diameter and zeta potential were measured by a dynamic light scattering method. This measurement was carried out at 25°C using Zetasizer Nano ZS, by charging 0.75 ml of the dispersion liquid of polyethylene glycol-bound titanium oxide conjugated particles into a zeta potential measuring cell and setting various parameters of the solvent to the same values as those for water. As a result of cumulant analysis, the dispersed particle diameter was found to be 54.2 nm.

### Example 2: Preparation of polyacrylic acid-bound titanium oxide conjugated particles

In the same manner as in Example 1, an acidic titanium oxide sol of a final solid content of 20% was prepared.

This acidic titanium oxide sol (0.6 ml) was dispersed in dimethylformamide (DMF) with a total volume adjusted to 20 ml. To this was added 10 ml of DMF, in which 0.3 g of polyacrylic acid of an average molecular weight of 5000 (manufactured by Wako Pure Chemical Ind., Ltd.) was dissolved, followed by mixing by stirring. The solution was transferred to a hydrothermal reaction vessel, HU-50 (manufactured by San-Ai Science Co., Ltd.), and a reaction was carried out at 150°C for 5 hours. After completion of the reaction, the reaction solution was cooled until the temperature of the reaction vessel became 50°C or lower and thereto was added isopropanol in a volume doubling the reaction solution. The mixture was allowed to stand at room temperature for 30 minutes and, thereafter, was centrifuged at 2000 g for 15 minutes to recover the precipitates. The surface of the recovered precipitates was washed with ethanol, and 1.5 ml of water was added thereto to obtain a dispersion liquid of polyacrylic acid-bound titanium oxide conjugated particles. This dispersion liquid was diluted with distilled water by a factor of 100, and the dispersed particle diameter and zeta potential were measured by a dynamic light scattering method. This measurement was carried out at 25°C using Zetasizer Nano ZS by charging 0.75 ml of the dispersion liquid of the polyacrylic acid-bound titanium oxide conjugated particles in a zeta potential measuring cell, and setting various parameters of the solvent to the same values as those for water. As a result, the dispersed particle diameter and the zeta potential were found to be 53.6 nm and -45.08 mV, respectively.

### Example 3: Preparation of polyethylene imine-bound titanium oxide conjugated particles

In the same manner as in Example 1, an acidic titanium oxide sol of a final solid content of 20% was prepared.

The titanium oxide sol thus obtained (3 ml) was dispersed in 20 ml of dimethylformamide (DMF) and to this was added 10 ml of DMF, in which 450 mg of polyethyleneimine having an average molecular weight of 10000 (manufactured by Wako Pure Chemical Ind., Ltd.) was dissolved, followed by mixing by stirring. The solution was transferred to a hydrothermal reaction vessel, HU-50 (manufactured by San-Ai Science Co., Ltd.), and the reaction was carried out at 150°C for 5 hours. After completion of the reaction, the reaction solution was cooled so that the temperature of the reaction vessel became 50°C or lower and thereto was added acetone in a volume doubling the reaction solution. The mixture was allowed to stand at room temperature for 30 min and, thereafter, was centrifuged at 2000 g for 15 minutes to recover the precipitates. The surface of the recovered precipitates was washed with ethanol, and 1.5 ml of water was added thereto to obtain a dispersion liquid of polyethyleneimine-bound titanium oxide conjugated particles. This dispersion liquid was diluted with distilled water by a factor of 100, and the dispersed particle diameter and zeta potential were measured by a dynamic light scattering method. This measurement was carried out at 25°C using Zetasizer Nano ZS by charging 0.75 ml of the dispersion liquid of the polyethyleneimine-bound titanium oxide conjugated particles in a zeta potential measuring cell and setting various parameters of the solvent to the same values as those for water. As a result, the dispersed particle diameter and the zeta potential were found to be 57.5 nm and 47.5 mV, respectively.

### Example 4: Binding of dihydroxyphenylpropionic acid to titanium oxide conjugated particles

Titanium oxide conjugated particles obtained in Example 1 and dihydroxyphenylpropionic acid were mixed in ultrapure water according to the compositions shown in Table 1 and adjusted to a total volume of 1 ml. The compositions were designated as titanium oxide conjugated particles A to C, respectively.

**[Table 1]**

| Material | Titanium oxide conjugated particles A | Titanium oxide conjugated particles B | Titanium oxide conjugated particles C |
|---|---|---|---|
| Titanium oxide conjugated particles | 2.5 wt % | 2.5 wt % | 0.7 wt % |
| Dihydroxyphenylpropionic acid | 0.94 wt % | 0.09 wt % | 0.01 wt % |
| Ultrapure water | 96.56 wt % | 97.41 wt % | 99.29 wt % |
| Total | 100 wt% | 100 wt% | 100 wt% |

The solutions prepared were allowed to stand at room temperature for 4 hours. After the reaction was complete, increase in absorbance was observed when absorption spectra of the solutions in the visible light wavelength region were measured by a UV-visible spectrophotometer and, thus, dihydroxyphenylpropionic acid was thought to have bound. Further, a change in the amount of dihydroxyphenylpropionic acid was obtained by measuring the peak at an absorption wavelength of 214 nm by a photodiode array detector before and after the reaction, using capillary electrophoresis according to the following conditions:
- Apparatus: P/ACE MDQ (manufactured by Beckman Coulter, Inc.)
- Capillary: fused silica capillary 50 µm i.d. × 67 cm (effective length, 50 cm) (manufactured by Beckman Coulter, Inc.)
- Mobile phase: 50 mM boric acid buffer solution (pH 9.0)
- Voltage: 25 kV
- Temperature: 20°C
From the obtained change in the amount, the amount of dihydroxyphenylpropionic acid bound per mass of titanium oxide was as listed in Table2.

**[Table 2]**

| Material | Titanium oxide conjugated particles A | Titanium oxide conjugated particles B | Titanium oxide conjugated particles C |
|---|---|---|---|
| Bound amount of dihydroxyphenylpropionic acid (mol/titanium oxide-g) | 2.0 x 10⁻⁴ | 5.0 x 10⁻⁵ | 2.0 x 10⁻⁵ |

Further, 1 ml of this solution was subjected to a free fall-type buffer exchange column NAP-10 (manufactured by GE Healthcare Bioscience) and eluted with 1.5 ml of water to remove unreacted dihydroxyphenylpropionic acid. Removal of dihydroxyphenylpropionic acid was confirmed by capillary electrophoresis in the same manner as described above and the absence of free dihydroxyphenylpropionic acid was confirmed. From these results, preparation of dihydroxyphenylpropionic acid-bound titanium oxide conjugated particles (titanium conjugated particles A to C) was confirmed.

### Example 5: Binding of an antibody to dihydroxyphenylpropionic acid-bound titanium oxide conjugated particles

A solution of the titanium oxide conjugated B out of the dihydroxyphenylpropionic acid-bound titanium oxide conjugated particles obtained in Example 4 and 1-ethyl-3-(3-dimethylaminopropyl)carbodiimide hydrochloride (manufactured by Dojindo Laboratories) were mixed in ultrapure water so that the respective concentrations became 20 mg/ml and 80 mM. The mixed solution was allowed to react at room temperature for 10 minutes. Using a desalting column PD-10 (manufactured by GE Healthcare Bioscience), the solution was subjected to solution exchange with a 20 mM HEPES buffer solution (pH 7.4) to obtain a solution of particles having a concentration of 20 mg/ml as titanium oxide. To this was added an anti-human serum albumin (anti-HSA) monoclonal antibody (mouse IgG: MSU-304, manufactured by Cosmo Bio Co., Ltd.) prepared in the same buffer solution as above, so that its amount became 3 mg/ml to obtain a total of 1 ml solution. After reaction at 4°C for 24 hours, ethanolamine was added to make a final concentration of 0.5 M and the solution was further reacted at 4°C for 1 hour. This solution was adjusted to a titanium oxide concentration of 1 mg/ml. When 1 ml of this solution was subjected to HPLC [AKTA purifier (manufactured by GE Healthcare Bioscience), column: Hiprep 16/60 Sephacryl S-500HR (manufactured by GE Healthcare Bioscience), mobile phase: phosphate buffered saline (pH 7.4), flow rate: 0.3 ml/min], UV absorption peaks were observed in a pass-through fraction and a fraction wherein an anti-HSA monoclonal antibody was confirmed to be present as a single component. These fractions were recovered. The pass-through fraction was thought, from the size of a molecule which was separated, to be a solution containing titanium oxide-antibody conjugated particles to which antibody molecules were bound. Also, the fraction wherein an anti-HSA monoclonal antibody was confirmed to be present as a single component was subjected to measurement of the protein concentration by Bradford method to confirm decrease in the antibody concentration before and after the reaction. From these results, it was confirmed that the titanium oxide-antibody conjugated particles could be prepared by binding an antibody to dihydroxyphenylpropionic acid-bound titanium oxide conjugated particles via dihydroxyphenylpropionic acid.

### Example 6: Binding of a fluorescent dye to titanium oxide-antibody conjugated particles

The titanium oxide-antibody conjugated particles, obtained in Example 5, were made into a dispersion liquid of 1% solid content using ultrapure water. Then, a solution of dopamine hydrochloride (molecular weight, Mn = 153.178: manufactured by Wako Pure Chemical Ind., Ltd.) was prepared so that its concentration became 200 mM. The solution prepared and the dispersion liquid were mixed in a 1:9 ratio to make a 1 ml solution and a binding reaction was carried out at room temperature for 4 hours. When an absorption spectrum of the solution after the reaction was measured in the visible light wavelength region by a UV-visible spectrophotometer, there was observed an increase in the absorbance in each solution. Thus, dopamine was thought to have bound. Further, solutions before and after the reaction were subjected to capillary electrophoresis according to the following conditions and a change in the amount of dopamine was obtained by measuring the peak at an absorption wavelength of 214 nm by a photodiode array detector:
- Apparatus: P/ACE MDQ (manufactured by Beckman Coulter, Inc.)
- Capillary: fused silica capillary 50 µm i.d. × 67 cm (effective length, 50 cm) (manufactured by Beckman Coulter, Inc.)
- Mobile phase: 50 mM sodium acetate buffer solution (pH 4.8)
- Voltage: 25 kV
- Temperature: 20°C
From the obtained change in the amount, the amount of dopamine bound per mass of titanium oxide was 4.0 × 10⁻⁵ dopamine-g/titanium oxide-g. From this result, the amount of linker molecules as a whole was 9.0 × 10⁻⁵ linker molecule-mol/titanium oxide particles-g.

Furthermore, 1.0 ml of this solution was subjected to a free fall-type buffer exchange column NAP-10 (manufactured by GE Healthcare Bioscience) and eluted with 1.5 ml of water to remove unreacted dopamine. Removal of dopamine was confirmed by capillary electrophoresis in the same manner as described above and the absence of unreacted dopamine was confirmed. From these results, preparation of dopamine-bound titanium oxide conjugated particles was confirmed. Then, this dopamine-bound titanium oxide-antibody conjugated particles and NHS-Rhodamine (manufactured by Pierce) were mixed in a 20 mM boric acid buffer solution so that their final concentrations were adjusted to be 0.3% and 1 mM, respectively. This solution was allowed to stand at 4°C by shutting off the light for 24 hours. This solution in the amount of 2.5 ml was subjected to a free fall-type buffer exchange column PD-10 (manufactured by GE Healthcare Bioscience) and eluted with 3.5 ml of water to remove the unreacted NHS-Rhodamine. Removal of unreacted NHS-Rhodamine was confirmed by capillary electrophoresis in the same manner as described above and the absence of free NHS-Rhodamine was confirmed. The solution obtained was subjected to spectral analysis by a fluorescent spectrophotometer and it was confirmed that the solution showed fluorescence at an excitation wavelength of 555 nm and a fluorescence wavelength of 575 nm. From these results, preparation of fluorescent molecule-bound titanium oxide-antibody conjugated particles was confirmed, wherein a fluorescent molecule is bound to the dopamine-bound titanium oxide-antibody conjugated particles via dopamine.

### Example 7: Evaluation of dispersibility of titanium oxide

### conjugated particles

The titanium oxide conjugated particles obtained in Example 1 (designated as titanium conjugated particles D) and the titanium oxide conjugated particles A to C obtained in Example 4 were each added to phosphate buffered saline so that the solid content became 0.05%. The solutions were allowed to stand at room temperature for 1 hour. Thereafter, the dispersed particle diameters and zeta potentials were measured using Zetasizer Nano ZS in the same manner as in Example 1. The results are shown in Table 3. Among titanium oxide conjugated particles A to D, it was confirmed that there was no big change in the dispersed particle diameter and zeta potential.

**[Table 3]**

| Material | Titanium oxide conjugated particles A | Titanium oxide conjugated particles B | Titanium oxide conjugated particles C | Titanium oxide conjugated particles D |
|---|---|---|---|---|
| Dispersed particle diameter (nm) | 54.4 | 53.9 | 54.5 | 54.2 |
| Z-potential (mV) | -3.71 | -6.87 | -7.43 | -7.21 |

### Example 8: Evaluation of dispersibility of titanium oxide-antibody conjugated particles

The titanium oxide-antibody conjugated particles obtained in Example 5 were added to phosphate buffered saline so that the solid content became 0.05%. The solution was allowed to stand at room temperature for 1 hour. Thereafter, the dispersed particle diameter and zeta potential were measured using Zetasizer Nano ZS in the same manner as in Example 1. As a result, the dispersed particle diameter was 52.5 nm and zeta potential was -4.48 mV. Thus, it was confirmed that there was no big difference compared to the results of Example 7.

### Example 9: Evaluation of singlet oxygen generation ability of titanium oxide conjugated particles induced by ultrasonic irradiation

The titanium oxide conjugated particles obtained in Example 1 (designated as titanium conjugated particles D) and titanium oxide conjugated particles A to C obtained in Example 4 were each added to phosphate buffered saline and the concentration was adjusted to a solid content of 0.05%. Also, as a control, phosphate buffered saline alone was prepared. To 3 ml each of the solutions, there was mixed, according to a manual, Singlet Oxygen Sensor Green Reagent (Molecular Probes Inc.), a reagent for measuring generation of singlet oxygen, to be used as the test solutions. The solutions were subjected to ultrasonic irradiation by an ultrasonic irradiation apparatus (manufactured by OG Giken Co., Ltd.; ULTRASONIC APPARATUS ES-2: 1 MHz) for 3 minutes at 0.4 W/cm² and 50% duty cycle operation. As samples for measurement, 400 µl each was withdrawn before and after the irradiation. For each sample, the fluorescence intensity at Ex=488 nm and Em=525 nm, due to generation of singlet oxygen, was measured by a fluorescence spectrophotometer (RF-5300PC; manufactured by Shimadzu Corporation). The results were as shown in Fig. 2. As shown in Fig. 2, it was confirmed that the titanium oxide conjugated particles A to D generated singlet oxygen more efficiently compared to the control. Also, it was thought that, as the amount of the linker bound per mass of the titanium oxide particles increased, generation of singlet oxygen was more suppressed.

### Example 10: Evaluation of binding of titanium oxide-antibody conjugated particles to antigen

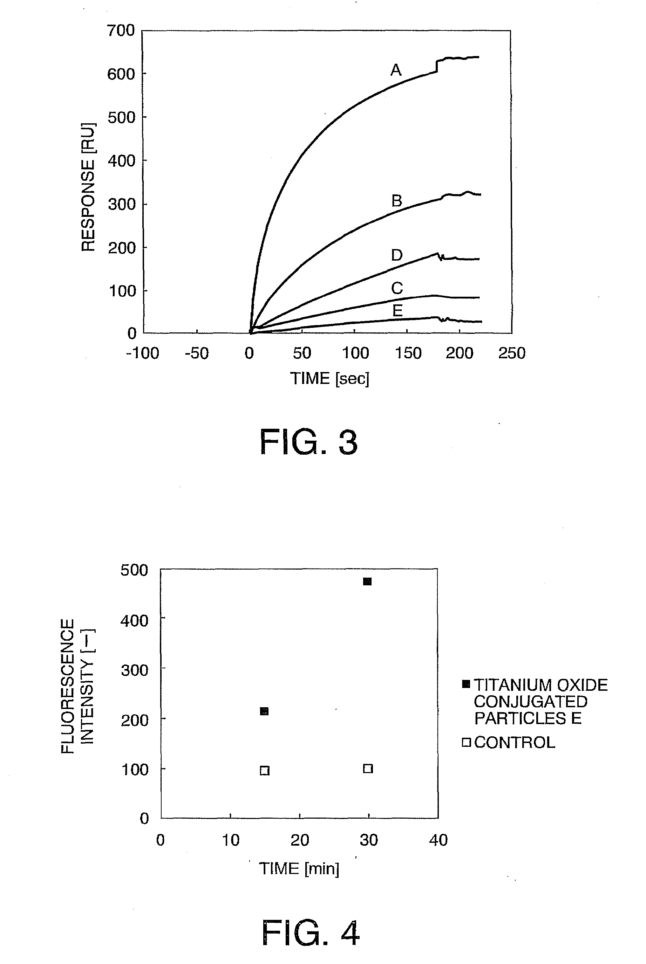

In order to confirm binding of the titanium oxide-antibody conjugated particles to an antigen by an SPR sensor, a sensor chip C1 (manufactured by Biacore) was set on an SPR sensor apparatus (BIACORE 1000, manufactured by Biacore) and a reaction to immobilize human serum albumin (HSA: manufactured by Wako Pure Chemical Ind., Ltd.) thereon was carried out according to the manufacturer's manual. The carboxyl group on the surface of the sensor chip was succinylated by flowing 50 µl of an NHS-EDC mixed solution, which was included in the BIAcore amine coupling kit (manufactured by Biacore), at a rate of 5 µl/min. Thereafter, the reaction was carried out by loading 50 µl of a solution of HSA, which was dissolved in a 10 mmol/l acetic acid-sodium acetate buffer solution (pH 5.0) in a concentration of 1 g/l, at a flow rate of 5 µl/min. After the reaction was complete, 50 µl of 1 mol/l ethanolamine, included in the BIAcore amine coupling kit, was loaded at a flow rate of 5 µl/min to carry out a blocking treatment of the succinyl group which did not participate in the binding. In this way, binding of HSA in an amount of about 0.8 ng/mm² was obtained. Then, 60 µl each of the titanium oxide-antibody conjugated particles obtained in Example 5 and anti-human serum albumin (anti-HSA) monoclonal antibody (mouse IgG: MSU-304, manufactured by Cosmo Bio Co., Ltd.), concentrations of which were adjusted to the respective values, were loaded at a flow rate of 30 µl/min. After confirming a reaction on the sensorgram, 30 µl of a 100 mmol/l glycine-NaOH buffer solution (pH 12.0) was loaded at a rate of 30 µl/min to carry out a dissociation reaction from the sensor. Analysis of the sensorgram was carried out by using Biomolecular Interaction Analysis (BIA) evaluation software (version 3.5, produced by Biacore). As a background, the result obtained by loading in the same way the titanium oxide conjugated particles, obtained in Example 1, was subtracted. The results were as shown in Fig. 3. Therein, the symbols represent the following, respectively: A; 0.05% titanium oxide-antibody conjugated particles, B; 0.005% titanium oxide-antibody conjugated particles, C; 0.0005% titanium oxide-antibody conjugated particles, D; 5 µg/ml anti-human serum albumin (anti-HSA) monoclonal antibody, E; 1 µg/ml anti-HSA monoclonal antibody. From these results, it was shown that the titanium oxide-antibody conjugated particles bound strongly to the antigen.

### Example 11: Binding of dihydroxyphenylpropionic acid to titanium oxide conjugated particles 2

Titanium oxide conjugated particles obtained in Example 1 and hydroxyphenylpropionic acid were mixed in 1) a 20 mmol/l acetic acid- sodium acetate buffer solution (pH = 3.6), 2) a 20 mmol/l MES buffer solution (manufactured by Dojindo Laboratories; pH = 6.0), and 3) a 20 mmol/l HEPES buffer solution (manufactured by Dojindo Laboratories; pH = 8.1) to prepare 0.8 ml solutions, where the final concentrations of titanium oxide conjugated particles and dihydroxyphenylpropionic acid were adjusted to 2% and 50 mmol/l, respectively.

The solutions prepared were stirred at 40°C for 25 hours. The absorption spectrum of each solution in the UV to visible light wavelength region (200 to 600 nm) was measured by a UV-visible spectrophotometer. Regarding a solution wherein only dihydroxyphenylpropionic acid was mixed, there was almost no spectral change in 1) a 20 mmol/l acetic acid-sodium acetate buffer solution (pH = 3.6). In contrast, in 2) a 20 mmol/l MES buffer solution ((pH = 6.0), and 3) a 20 mmol/l HEPES buffer solution (pH = 8.1), changes in the absorption spectra were confirmed compared to 0 hour after preparation and there was also observed a color change into light red by visual observation. From these results, dihydroxyphenylpropionic acid was thought to undergo a change and be unstable at a pH = 6.0 or higher. Further, regarding a solution wherein titanium oxide conjugated particles and dihydroxyphenylpropionic acid were mixed, in 1) a 20 mmol/l acetic acid-sodium acetate buffer solution (pH = 3.6), there was observed a change in the absorption spectrum compared to 0 hour after preparation and there was also observed a color change into dark brown by visual observation. Because there was no big change with dihydroxyphenylpropionic acid alone, this change was thought to be due to the occurrence of charge transfer by the binding of dihydroxyphenylpropionic acid to the titanium oxide conjugated particles.

Then, in 1) a 20 mmol/l acetic acid-sodium acetate buffer solution (pH = 3.6), solutions at 0 hour after preparation and after stirring for 25 hours were subjected to capillary electrophoresis according to the following conditions and a change in the amount of dihydroxyphenylpropionic acid was obtained by measuring the peak at an absorption wavelength 214 nm by a photodiode array detector:
- Apparatus: P/ACE MDQ (manufactured by Beckman Coulter, Inc.)
- Capillary: fused silica capillary 50 µm i.d. × 67 cm (effective length, 50 cm) (manufactured by Beckman Coulter, Inc.)
- Mobile phase: 50 mM boric acid buffer solution (pH 9.0)
- Voltage: 25 kV
- Temperature: 20°C
From the change in the amount obtained, the amount of dihydroxyphenylpropionic acid bound per mass of titanium oxide in 1) a 20 mmol/l acetic acid-sodium acetate buffer solution (pH = 3.6) was 7.7 × 10⁻⁴ dihydroxyphenylpropionic acid-mol/titanium oxide particles-g.

### Example 12: Test of cell killing induced by ultrasonic irradiation

The titanium oxide-antibody conjugated particles obtained in Example 5 were added in an amount of 1/10 to 3 ml of a 10% serum-added RPMI 1640 medium (manufactured by Invitrogen) containing 1 × 10⁵ cells/ml Jurkat cells and adjusted to prepare test solutions having final the concentrations of 0.05% and 0%. Each of the test solutions was subjected to ultrasonic irradiation using an ultrasonic irradiation apparatus (manufactured by OG Giken Co., Ltd.; ULTRASONIC APPARATUS ES-2: 1 MHz) for 15 seconds (0.5W/cm² and 50% pulse). The number of cells were measured by the MTT assay (manufactured by Dojindo Laboratories) according to the manufacturer's procedure manual, and a cell survival rate was calculated in terms of the number of cells before the test being set as 100%. As a result, at a final concentration of 0.05%, the survival rate was 75.8%. Also, at the final concentration of 0%, the cell survival rate was 99.2%. From these results, the cell killing effect of titanium oxide-antibody conjugated particles induced by ultrasonic irradiation was confirmed.

### Example 13: Binding of ferrocenecarboxylic acid and dopamine to titanium oxide conjugated particles

Ferrocenecarboxylic acid (manufactured by Wako Pure Chemical Ind., Ltd.) and dopamine hydrochloride (manufactured by Wako Pure Chemical Ind., Ltd.) were each dissolved in dimethylformamide (DMF; manufactured by Wako Pure Chemical Ind., Ltd.) in the concentration of 1 mM. Also, similarly by using DMF, solutions containing 200 mM benzotriazol-1-yl-oxy-tris(pyrrolidino)phosphonium hexafluorophosphate (PyBop; manufactured by Merck KGaA), 200 mM 1-hydroxybenzotriazole (HoBt; manufactured by Dojindo Laboratories), and 20 mM N,N-diisopropylethylamine (DIEA; manufactured by Wako Pure Chemical Ind., Ltd.) were prepared, respectively. These were mixed and adjusted into a solution of 20 ml with DMF, whereby the concentrations of ferrocenecarboxylic acid and dopamine hydrochloride were adjusted to 1/4 of the original concentrations and the concentrations of other components were adjusted to 1/10 of the original concentrations. This mixed solution was reacted at room temperature for 20 hours under gentle stirring.

A portion of the reaction mixture was diluted 10 times with ultrapure water and the resultant solution was analyzed by reverse-phase chromatography (HPLC system: Prominence (manufactured by Shimadzu Corporation), column: Chromolith RP-18e 100-3 mm (manufactured by Merck KGaA), mobile phase: A) methanol (manufactured by Wako Pure Chemical Ind., Ltd.); B) 0.1% aqueous trifluoroacetic acid solution (manufactured by Wako Pure Chemical Ind., Ltd.), flow rate: 2 ml/min). Using a UV detector set at a wavelength of 210 nm, gradient elution was carried out in such a way that the mobile phase became 100% methanol in 1 to 10 minutes after injection (0.02 ml). As a result, there was observed a peak which was thought to be due to a conjugate formed between ferrocenecarboxylic acid and dopamine hydrochloride. Also, peaks due to ferrocenecarboxylic acid and dopamine hydrochloride by themselves were below detection limits. From these results, formation of a conjugate between ferrocenecarboxylic acid and dopamine hydrochloride was confirmed.

The remainder of the reaction mixture was concentrated 10 times under reduced pressure to prepare a concentrated reaction solution. Titanium oxide conjugated particles obtained in Example 1 were adjusted with ultrapure water to a solution with a solid content of 1% and, thereto was mixed the concentrated reaction solution in a 1/10 amount to prepare a total of 1 ml solution. Under gentle stirring, this mixed solution was allowed to react at room temperature for 1 hour. After the reaction was complete, a precipitated component was removed by centrifugal separation (1500 g, 10 min) to recover the supernatant liquid. This solution (1 ml) was subjected to a free fall-type buffer exchange column NAP-10 (manufactured by GE Healthcare Bioscience) and eluted with 1.5 ml of water to remove the unreacted conjugate between ferrocenecarboxylic acid and dopamine hydrochloride, as well as DMF. When an absorption spectrum of this solution in visible light region (400 nm) was measured by a UV-visible spectrophotometer, increase in absorbance was observed, and thus the conjugate between ferrocenecarboxylic acid and dopamine hydrochloride was thought to have bound. From these results, preparation of titanium oxide conjugated particles having a conjugate between ferrocenecarboxylic acid and dopamine hydrochloride bound thereto was confirmed.

### Example 14: Binding of ferrocenecarboxylic acid and dopamine to titanium oxide-antibody conjugated particles

Titanium oxide-antibody conjugated particles having a conjugate between ferrocenecarboxylic acid and dopamine hydrochloride bound thereto was prepared in the same manner as in Example 13, except that titanium oxide-antibody conjugated particles obtained in Example 5 were used instead of the titanium oxide conjugated particles obtained in Example 1.

### Example 15: Evaluation of ultrasonic wave-induced hydroxyl radical generation of titanium oxide conjugated particles having a conjugate between ferrocenecarboxylic acid and dopamine hydrochloride bound thereto

The titanium oxide conjugated particles having a conjugate between ferrocenecarboxylic acid and dopamine hydrochloride bound thereto (designated as titanium conjugated particles E), obtained in Example 13, was added to phosphate buffered saline (pH 7.4) and adjusted so that the solid content became 0.05%. In addition, as a control, phosphate buffered saline (pH 7.4) alone was used. Each 3 ml solution was prepared as the test solution. Each solution was subjected to ultrasonic irradiation by an ultrasonic irradiation apparatus (manufactured by OG Giken Co., Ltd.; ULTRASONIC APPARATUS ES-2: 1 MHz) for 3 minutes (0.4 W/cm² and 50% pulse). After irradiation, hydroxyphenyl fluorescein (HPF, manufactured by Daiichi Pure Chemicals Co., Ltd.) was mixed to each solution according to a manual and the mixture was allowed to stand at room temperature for 15 minutes and 30 minutes. As the test samples at each standing time, 400 µl each was withdrawn from each solution before and after irradiation. With each sample, fluorescence intensity at Ex=490 nm and Em=515 nm due to generation of hydroxy radicals were measured by a fluorescence spectrophotometer (RF-5300PC; manufactured by Shimadzu Corporation). The results were as shown in Fig. 4. As shown in Fig. 4, it was confirmed that titanium oxide conjugated particles E generate hydroxyl radicals more efficiently compared to the control. Also, it was thought that the hydroxyl radicals were continually generated, because the titanium oxide conjugated particles E showed increased fluorescent intensity with time of standing.

### Example 16: Binding of an antibody to titanium oxide conjugated particles via dopamine

After adjusting 0.1 mg of anti-α-fetoprotein (anti-AFP) antibody (mouse IgG: NB-013, manufactured by Nippon Biotest Laboratories, Inc.) with coupling buffer (pH 5.5; Catalog No. 153-6054, manufactured by Bio-Rad Laboratories, Inc.) to a 1.8 ml volume, 0.2 ml of an aqueous solution of sodium periodate (manufactured by Wako Pure Chemical Ind., Ltd.) was added thereto in a concentration of 25 mg/1.2 ml and the resultant solution was reacted at room temperature for 1 hour. Thereafter, ultrapure water was added thereto to make a solution of 2.5 ml, which was subjected to a free fall-type buffer exchange column PD-10 (manufactured by GE Healthcare Bioscience) and eluted with 3.2 ml of a 20 mM MES buffer solution (pH 5.5) to remove unreacted sodium periodate. The solution was centrifuged (1500 g, 15 min) by Amicon Ultra-15 (MWCO = 5000; manufactured by Millipore Corporation) and concentrated to a 0.7 ml volume to obtain an oxidized antibody solution. Also, 0.5 ml of an aqueous 200 mM solution of dopamine hydrochloride (manufactured by Wako Pure Chemical Ind., Ltd.) and 0.1 ml of an aqueous 70 mM solution of succinimidyl 4-hydrazinonicotinate acetone hydrazone (SANH; manufactured by Pierce) were mixed and adjusted to a total of 1 ml volume with a 100 mM HEPES buffer solution (pH 8.1) and ultrapure water. The solution was allowed to react at room temperature for 1 hour, and binding of SANH and dopamine was confirmed by thin-layer chromatography. Thus, a solution of a conjugate between SANH and dopamine was obtained. These, namely, 0.7 ml of the oxidized antibody solution, 0.1 ml of a solution of a conjugate between SANH and dopamine, and 0.2 ml of coupling buffer (pH 5.5; Catalog No. 153-6054, manufactured by Bio-Rad Laboratories, Inc.) were mixed and reacted at 4°C for 16 hours. Thereafter, the solution was subjected to a free fall-type buffer exchange column PD-10 (manufactured by GE Healthcare Bioscience), and eluted with a PBS buffer solution (phosphate buffer saline) to remove a conjugate between antibody, SANH, and dopamine, and an unreacted conjugate between SANH and dopamine. In this way, a conjugate between antibody, SANH, and dopamine was obtained. Then, 2 ml of 5% (wt/vol) titanium oxide conjugated particles obtained in Example 1 and 1 ml of a conjugate between antibody, SANH, and dopamine were mixed and reacted at 4°C for 16 hours. Thereafter, using 44.5 mm PBCC membrane (MWCO = 300000; Catalog No. PBMK 04310, manufactured by Millipore Corporation) and Stirred Cell Model 8050 (Catalog No. 5122, manufactured by Millipore Corporation), an unbound conjugate between the antibody, SANH, and dopamine were removed accompanying a solution exchange of 334 ml under 10 psi according to the manufacturer's protocol. In this way, a dispersion liquid of titanium oxide-anti-AFP antibody conjugated particles was obtained.

Next, in order to confirm binding of the titanium oxide-anti-AFP antibody conjugated particles to an antigen by an SPR sensor, a sensor chip C1 (manufactured by Biacore) was set on an SPR sensor apparatus (BIACORE 1000, manufactured by Biacore) and a reaction to immobilize α-fetoprotein (AFP: manufactured by Nippon Biotest Laboratories, Inc.) thereon was carried out according to the manufacturer's manual. As the mobile phase, a mixed solution based on phosphate buffer (10 mM phosphate buffer solution (pH 7.4), 150 mM NaCl, 0.05% (wt/vol) Tween 20) was used. The carboxyl group on the surface of the sensor chip was succinylated by flowing 200 µl of an NHS-EDC mixed solution which was included in the BIAcore amine coupling kit (manufactured by Biacore) at a rate of 30 µl/min. Thereafter, a reaction was carried out by loading 180 µl of a solution of AFP, dissolved in 90 mmol/l acetic acid-sodium acetate buffer solution (pH 5.0) in a concentration of 100 µg/ml, at a flow rate of 30 µl/min. After the reaction was complete, 150 µl of 1 mol/l ethanolamine, included in the BIAcore amine coupling kit, was loaded at a flow rate of 30 µl/min to carry out a blocking treatment of the succinyl group which did not participate in the binding. In this way, a response of about 150 RU was obtained. A mixed solution (90 µl) of the 0.025% (wt/vol) dispersion liquid of titanium oxide-anti-AFP antibody conjugated particles prepared and 10 µg/ml anti-α-fetoprotein (anti-AFP) antibody (mouse IgG: NB-013, manufactured by Nippon Biotest Laboratories, Inc.), adjusted to the respective concentrations, were loaded at a flow rate of 30 µl/min. After confirming a reaction on the sensorgram, 100 µl of a 100 mmol/l glycine-NaOH buffer solution (pH 12.0) was loaded at 30 µl/min to carry out a dissociation reaction from the sensor. Analysis of the sensorgram was conducted by using Biomolecular Interaction Analysis (BIA) evaluation software (version 3.5, produced by Biacore). As a background, the result obtained by loading in the same way the titanium oxide conjugated particles, obtained in Example 1, was subtracted. As a result, a response of 15 RU was obtained in the case of anti-AFP and a response of 50 RU was obtained in the case of titanium oxide-anti-AFP antibody conjugated particles. From these results, it was shown that the titanium oxide-anti-AFP antibody particles bound strongly to the antigen. From above, it was confirmed that titanium oxide-antibody conjugated particles was prepared, wherein an anti-AFP antibody bound to titanium oxide conjugated particles via dopamine.

## Claims

1. An antitumor agent comprising titanium oxide-antibody conjugated particles which exhibit catalytic activity upon ultrasonic irradiation, comprising:
titanium oxide conjugated particles comprising titanium oxide particles and a water-soluble polymer bound to the surface of the titanium oxide particles via at least one functional group selected from a group consisting of a carboxyl group, an amino group, a diol group, a salicylic acid group, and a phosphoric acid group;
a linker molecule further bound to the surface of the titanium oxide conjugated particles,
the linker molecule being a compound:
(1) having at least one functional group selected from a group consisting of a carboxyl group, an amino group, a diol group, a salicylic acid group, and a phosphoric acid group and
(2) having a) a saturated or unsaturated chain hydrocarbon group having 6 to 40 carbon atoms, b) a substituted or unsubstituted, saturated or unsaturated 5- or 6-membered heterocyclic group, or c) a substituted or unsubstituted, saturated or unsaturated 5- or 6-memebered cyclic hydrocarbon group, and
the linker molecule being bound to the titanium oxide via the functional group without polymerization of the functional groups with each other; and
an antibody being further bound to the titanium oxide via the linker molecule.

2. The antitumor agent according to claim 1, wherein an amount of the linker molecule bound per mass of the titanium oxide particles is 1.0 × 10⁻⁶ to 1.0 × 10⁻³ mol/titanium oxide particles-g.

3. The antitumor agent according to claim 1, wherein the linker molecule is a catechol, preferably at least one kind selected from a group consisting of dopamine and dihydroxyphenylpropionic acid.

4. The antitumor agent according to claim 1, wherein the water-soluble polymer has a weight average molecular weight of 5000 to 40000.

5. The antitumor agent according to claim 1, wherein the water-soluble polymer contains at least one selected from a group consisting of polyethylene glycol, polyacrylic acid, and polyethyleneimine.

6. The antitumor agent according to claim 1, wherein the titanium oxide particles are particles of anatase-type titanium oxide or rutile-type titanium oxide.

7. The antitumor agent according to claim 1, having a particle size of 20 to 200 nm.

8. The antitumor agent according to claim 1, wherein a fluorescent molecule is further bound via the linker molecule.

9. The antitumor agent according to claim 1, wherein a molecule containing a low-valent transition metal is further bound via the linker molecule.

10. The antitumor agent according to claim 1, wherein the antitumor agent kills cancer cells upon activation by ultrasonic or ultraviolet irradiation.

11. A dispersion liquid comprising the antitumor agent according to claim 1 and a solvent in which the antitumor agent is dispersed.

12. The dispersion liquid according to claim 11, wherein the solvent is a water-based solvent.

13. The dispersion liquid according to claim 11, wherein pH of the solvent is 5 to 8.

14. The dispersion liquid according to claim 11, wherein the solvent is physiological saline.

15. The dispersion liquid according to claim 11, wherein the antitumor agent is contained in an amount of 0.001 to 1% by mass.
